# EUROPEAN PATENT APPLICATION

(11) **EP 2 901 927 A1**
(43) Date of publication of application: **05.08.2015**
(21) Application number: 15153023.5
(22) Date of filing: 29.01.2015
(51) Int. Cl.: A61B 5/145

(54) **Biological information measurement apparatus and biological information measurement method**

(30) Priority: 31.01.2014 JP 2014017024
(71) Applicant: Seiko Epson Corporation, Tokyo 163 (JP)
(72) Inventor: Kasahara, Hirokazu, Nagano, 392-8502 (JP); Mizobe, Kimitake, Nagano, 392-8502 (JP); Ishiguro, Hideto, Nagano, 392-8502 (JP)
(74) Representative: Hoffmann Eitle

(57) **Abstract**

A blood glucose level measurement apparatus is a non-invasive measurement apparatus which is mounted on the wrist or the like of a user and performs a measurement by using light. When a biological image is captured in order to acquire a blood vessel pattern, not all light emitting elements emit light (entire surface emission is not performed) but some of the light emitting elements within a light emission range emit light. The light emission range is set to a range centering on an irradiation position (measurement light emitting element) in the previous measurement.

## Description

### BACKGROUND

### 1. Technical Field

The present invention relates to a biological information measurement apparatus and the like which measure biological information related to a blood vessel or blood.

### 2. Related Art

As a measurement apparatus which measures biological information related to a blood vessel or blood in a non-invasive manner by using light, there is a blood glucose level measurement apparatus which measures a glucose concentration in blood, that is, a blood glucose level. A blood glucose level greatly varies in a day depending on a meal or exercise and is thus required to be frequently measured. Therefore, in order for a diabetic or the like himself/herself to measure and manage a blood glucose level, a portable blood glucose level measurement apparatus is desirable on the condition of its continuous use. However, the apparatus is portable and is thus required to operate by using a battery. From the viewpoint of the use time, or a frequency of charging or exchanging a battery, a large-capacity battery is desirable, but from the viewpoint of portability, a small-capacity battery which is small and light weight is desirable. In either viewpoint, reducing power consumption is an important technique.

As a technique for reducing power consumption, for example, JP-A-10-314150 discloses a technique in which measurement is intermittently performed in an apparatus which measures a blood component.

Meanwhile, the continuous measurement of a blood glucose level is aimed at rapidly detecting a low blood glucose state in which a blood glucose level is low. The low blood glucose state is a life-threatening and is thus required to be rapidly detected. In a case of employing the technique in which the measurement is intermittently performed as disclosed in JP-A-10-314150, if a measurement interval is set to be too long, there is a risk that detection of the low blood glucose state may be delayed. In order to rapidly detect the low blood glucose state, a measurement interval is preferably short, but, in this case, there is a problem in that power consumption increases. As a measurement target, a blood glucose level is exemplified, but the same problem may also occur in a measurement of biological information related to a blood vessel or the blood.

### SUMMARY

An advantage of some aspects of the invention is to reduce power consumption in a biological information measurement apparatus which measures biological information related to a blood vessel or blood.

A first aspect of the invention is directed to a biological information measurement apparatus including an optical sensor unit that includes a light emitting unit in which a plurality of light emitting elements are disposed in a planar shape, and a light receiving unit receiving light which is a result of light emitted by the light emitting unit being reflected or transmitted inside a living body; a light emission range setting unit that sets a light emission range of the light emitting unit; a blood vessel position detecting unit that causes the light emitting elements within the light emission range to emit light and detects a blood vessel position on the basis of an image obtained by the light receiving unit receiving the light; and a measurement unit that controls light emission of the light emitting elements selected on the basis of the detected blood vessel position, and light reception in the light receiving unit, so as to measure biological information related to a blood vessel or blood, in which the light emission range setting unit sets the light emission range on the basis of past measurement information obtained by the measurement unit.

As another aspect of the invention, the first aspect may be configured as a biological information measurement method including setting a light emission range of a light emitting unit of an optical sensor unit that includes the light emitting unit in which a plurality of light emitting elements are disposed in a planar shape, and a light receiving unit receiving light which is a result of light emitted by the light emitting unit being reflected or transmitted inside a living body; causing the light emitting elements within the light emission range to emit light and detecting a blood vessel position on the basis of an image obtained by the light receiving unit receiving the light; and controlling light emission of the light emitting elements selected on the basis of the detected blood vessel position, and light reception in the light receiving unit, so as to measure biological information related to a blood vessel or blood, in which the setting of the light emission range includes setting the light emission range on the basis of past measurement information obtained by measuring the biological information.

According to the first aspect and the like, a light emission range of the light emitting unit for acquiring a blood vessel position is set on the basis of past measurement information. In order to measure biological information related to a blood vessel or blood, first, a blood vessel position is required to be detected. According to the present aspect, a light emission range is set on the basis of past measurement information, and thus a blood vessel position can be highly reliably detected. Since a light emission range is set to be small, the number of light emitting elements to emit light is reduced, and thus it is possible to reduce power consumption.

As a second aspect of the invention, the biological information measurement apparatus according to the first aspect may be configured such that the light emission range setting unit sets the light emission range on the basis of a position of a light emitting element which is selected in a past measurement by the measurement unit.

According to the second aspect, the light emission range is set on the basis of a position of a light emitting element which emitted light in order to measure biological information in a past measurement. Consequently, it is possible to set a light emission range which allows a blood vessel position to be detected with high reliability.

As a third aspect of the invention, the biological information measurement apparatus according to the first or second aspect may be configured such that, in a case where there is no past measurement performed by the measurement unit, the light emission range setting unit sets, as the light emission range, a range which is wider than in a case where there is a past measurement.

According to the third aspect, in a case where there is no past measurement, the light emission range is set to a range which is wider than in a case where there is a past measurement. In other words, in a case where there is a past measurement, a light emission range is set to be narrower than in a case where there is no past measurement. Consequently, in a case where continuous measurements are performed, the number of light emitting elements to emit light is reduced in the second and subsequent measurements, and thus it is possible to reduce power consumption.

As a fourth aspect of the invention, the biological information measurement apparatus according to any one of the first to third aspects may be configured such that the biological information measurement apparatus further includes a re-execution control unit that causes the light emission range setting unit to reset a different range as the light emission range and the blood vessel position detecting unit to detect a blood vessel position again in a case where a blood vessel position is not detected by the blood vessel position detecting unit.

According to the fourth aspect, in a case where a blood vessel position is not detected, a range different from a set range is reset as the light emission range, and light emitting elements within the reset light emission range are made to emit light, so that a blood vessel position is detected again.

As a fifth aspect of the invention, the biological information measurement apparatus according to the fourth aspect may be configured such that the re-execution control unit causes the light emission range setting unit to reset a range which is wider than a previously set light emission range, as the light emission range.

According to the fifth aspect, the reset light emission range is wider than the set light emission range. Consequently, the light emission range is reset so as to increase a possibility that a blood vessel position may be detected.

### BRIEF DESCRIPTION OF THE DRAWINGS

The invention will be described with reference to the accompanying drawings, wherein like numbers reference like elements.
Fig. 1 is an exterior example of a blood glucose level measurement apparatus.
Figs. 2A and 2B are configuration diagrams of a sensor module.
Fig. 3 is a diagram for explaining acquisition of a biological image.
Fig. 4 is a diagram illustrating an example of a biological image.
Fig. 5 is a diagram for explaining acquisition of a blood vessel pattern.
Fig. 6 is a diagram for explaining selection of a measurement target blood vessel part.
Fig. 7 is a diagram for explaining selection of an irradiation position and a light reception position.
Fig. 8 is a diagram for explaining the optimum distance between the irradiation position and the light reception position.
Fig. 9 is a diagram for explaining a light emission range.
Fig. 10 is a diagram for explaining an imaging range based on the light emission range.
Fig. 11 is a diagram illustrating a functional configuration of a blood glucose level measurement apparatus.
Fig. 12 is a diagram illustrating a configuration example of blood vessel part data.
Fig. 13 is a flowchart illustrating a blood glucose level measurement process.

### DESCRIPTION OF EXEMPLARY EMBODIMENTS

### Entire Configuration

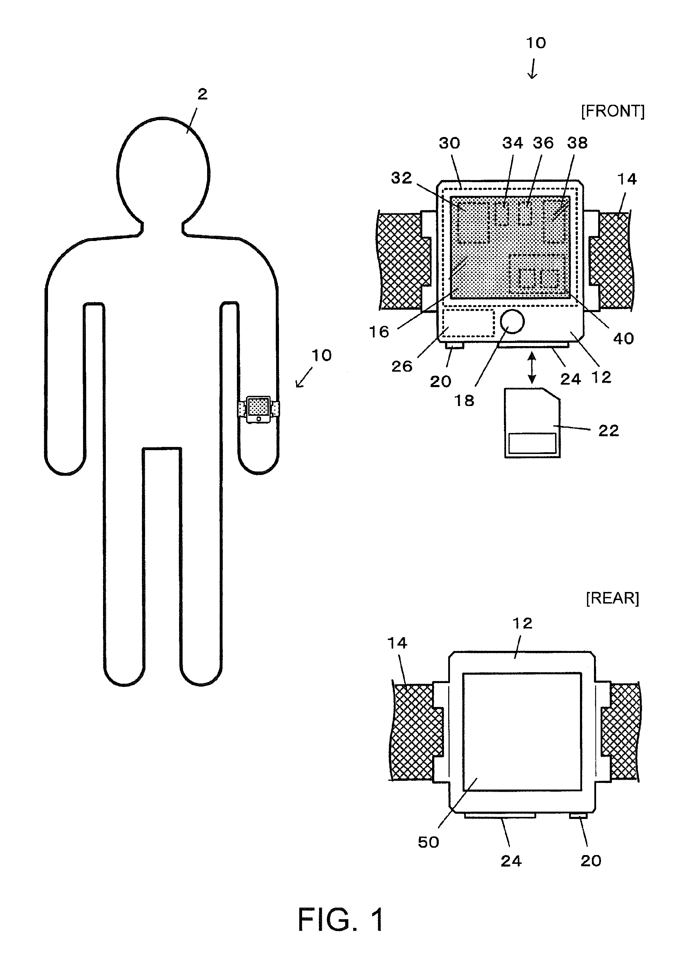

Fig. 1 is a diagram illustrating a configuration example of a blood glucose level measurement apparatus 10 of the present embodiment. The blood glucose level measurement apparatus 10 is a biological information measurement apparatus which measures biological information of a user 2 by using light in a non-invasive manner, and measures a blood glucose level which is a glucose concentration in blood of the user 2 as the biological information. As illustrated in Fig. 1, the blood glucose level measurement apparatus 10 is of a wearable apparatus which is a wrist watch type and includes a main body case 12, and a fixation band 14 such as Velcro tape (registered trademark) for mounting and fixing the main body case 12 on and to a measurement part such as the wrist or the arm of the user 2.

A touch panel 16 and an operation switch 18 are provided on a front surface (a surface directed outwards when the user 2 wears the blood glucose level measurement apparatus 10) of the main body case 12. The user 2 inputs a measurement start instruction by using the touch panel 16 or the operation switch 18, or a measurement result is displayed on the touch panel 16.

A communication device 20 for communication with external devices and a reader/writer 24 of a memory card 22 are provided on a side surface of the main body case 12. The communication device 20 is implemented by a jack for attaching and detaching a wired cable, or a wireless communication module and an antenna for performing wireless communication. The memory card 22 is a data rewritable nonvolatile memory such as a flash memory, a ferroelectric random access memory (FeRAM), or a magnetoresistive random access memory (MRAM).

A sensor module 50 is provided on a rear surface of the main body case 12 so as to be in contact with a skin surface of the user 2. The sensor module 50 is a measurement device which irradiates the skin surface of the user 2 with measurement light and receives reflected or transmitted light, and is a thin image sensor having a light source therein.

A charge type battery 26 and a control board 30 are built into the main body case 12. As a method of charging the battery 26, there may be a configuration in which an electric contact is provided on the rear surface side of the main body case 12, the main body case 12 is set in a cradle connected to a domestic power supply, and the battery 26 is charged via the electric contact and the cradle, or the battery 26 may be charged in a wireless manner.

The control board 30 is equipped with a central processing unit (CPU), a main memory, a measurement data memory, a touch panel controller, and a sensor module controller. The main memory is a storage medium which stores a program or initial setting data or stores calculation values of the CPU, and is implemented by a RAM, a read only memory (ROM), a flash memory, or the like. The program or the initial setting data may be stored in the memory card 22. The measurement data memory is a storage medium which stores measurement data, and is implemented by a data rewritable nonvolatile memory such as a flash memory, a ferroelectric random access memory (FeRAM), or a magnetoresistive random access memory (MRAM). The measurement data may be stored in the memory card 22.

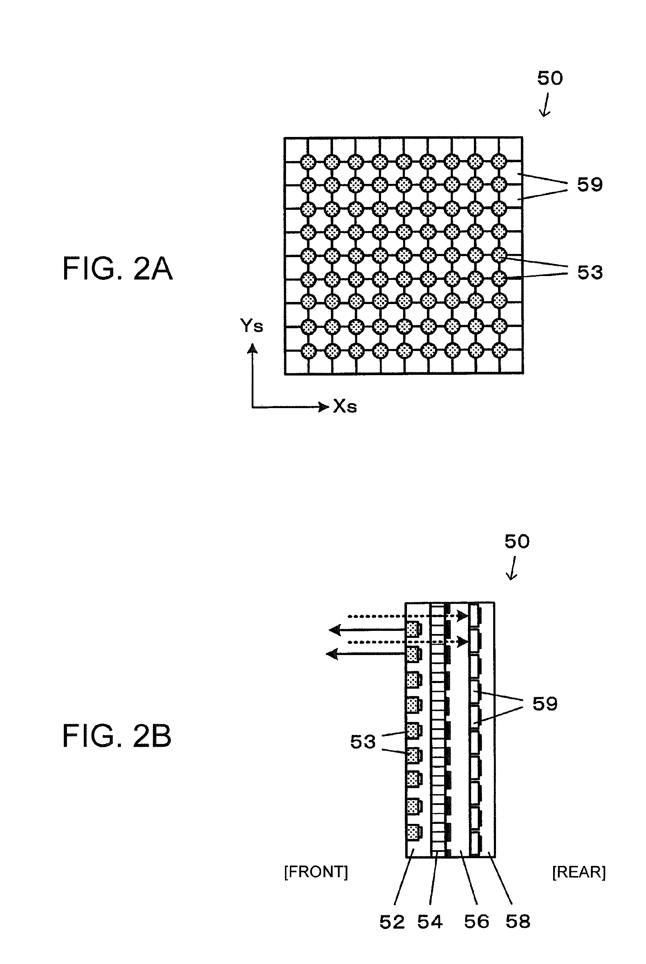

Figs. 2A and 2B are diagrams illustrating a configuration of the sensor module 50. Fig. 2A is a plan view and Fig. 2B is a cross-sectional view. The sensor module 50 is an optical sensor in which a light emitting layer 52, a light blocking layer 54, a spectroscopic layer 56, and a light receiving layer 58 are laminated. A plurality of light emitting elements 53 are arranged in a planar shape in a two-dimensional manner in the light emitting layer 52, and the light blocking layer 54 selectively blocks light which is not directed to the light receiving layer 58. The spectroscopic layer 56 selectively transmits near infrared rays therethrough, and a plurality of light receiving elements 59 are arranged in a planar shape in a two-dimensional manner in the light receiving layer 58. The sensor module 50 is provided on the rear side of the main body case 12 so that a front side thereof (a surface of the light emitting layer 52 side) is directed to the skin surface of the user 2.

The light emitting elements 53 are an irradiation unit which applies measurement light, and are implemented by, for example, light emitting diodes (LEDs), organic light emitting diodes (OLEDs), or the like. In the present embodiment, in order to measure a blood glucose level (a glucose concentration in blood), the light emitting elements 53 employ elements which can emit light including near infrared rays having subcutaneous transparency.

The light receiving elements 59 are a light receiving unit which receives transmitted light or reflected light of the measurement light and outputs an electric signal corresponding to a light reception amount, and are implemented by imaging elements such as charge coupled device (CCD) image sensors or complementary metal oxide semiconductor (CMOS) image sensors. A single light receiving element 59 includes a plurality of elements which receive wavelength components required for calibration.

The light emitting elements 53 of the light emitting layer 52 and the light receiving elements 59 of the light receiving layer 58 are disposed in a matrix which is defined in a common Xs-Ys orthogonal coordinate system. In addition, the light emitting elements 53 of the light emitting layer 52 and the light receiving elements 59 of the light receiving layer 58 are arranged at the same intervals in directions of the Xs and Ys axes but are alternately arranged in the Xs-Ys plane. In other words, positions of the light emitting elements 53 and the light receiving elements 59 in the directions of the Xs and Ys axes are laminated so as to be deviated relative to each other by a predetermined length. Consequently, light which has been transmitted through a biological tissue of the user 2 or light which has been reflected inside the biological tissue (hereinafter, the light is referred to as reflected/transmitted light as appropriate) can reach the light receiving elements 59.

An arrangement interval of each of the light emitting elements 53 in the light emitting layer 52 and the light receiving elements 59 in the light receiving layer 58 may be set as appropriate. For example, an arrangement interval of 1 to 500 [µm] is preferably used, and, for example, 50 to 200 [µm] may be used from the viewpoint of manufacturing cost and measurement accuracy. The light emitting elements 53 and the light receiving elements 59 are not only laminated but may also be arranged in parallel to each other.

### Principle

### (A) Measurement of blood glucose level

A description will be made of a measurement principle of a blood glucose level in the present embodiment. In order to perform a measurement, the blood glucose level measurement apparatus 10 is fixed with the fixation band 14 so that the sensor module 50 is brought into close contact with the skin surface of the user 2. Since the sensor module 50 is brought into close contact with the skin surface, it is possible to minimize factors which reduce measurement accuracy, such as reflection of measurement light at the skin surface or scattering thereof near the skin surface. A blood vessel in a biological tissue directly under the sensor module 50 is set as a measurement target, light including transmitted light which is a result of measurement light being transmitted through the blood vessel is received so as to obtain an absorption spectrum, and a blood glucose level is estimated and calculated.

### (A-1) Acquisition of blood vessel pattern

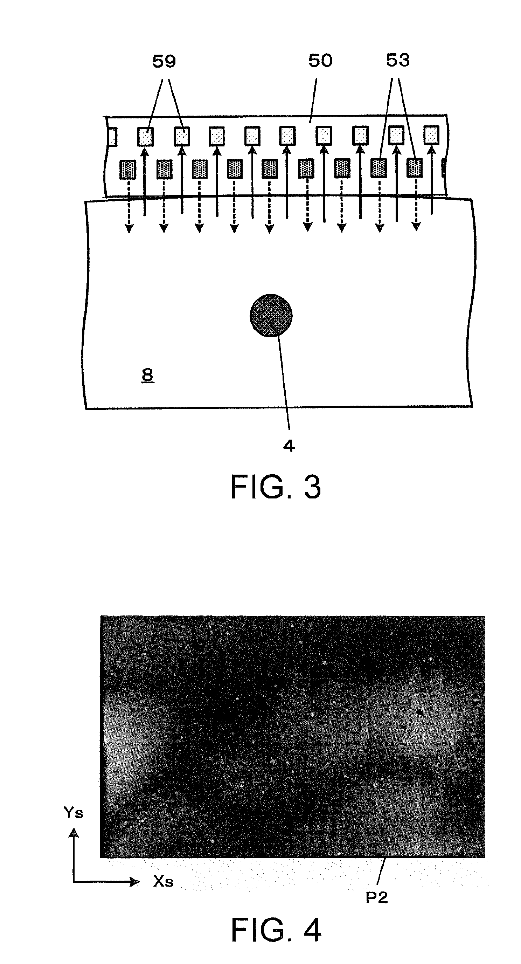

Specifically, first, a blood vessel pattern (a position of a blood vessel) which is viewed from the skin surface is acquired. The acquisition of a blood vessel pattern may be performed in the same manner as in the vein pattern detection of the well-known vein authentication technique. In other words, as schematically illustrated in Fig. 3, the light emitting elements 53 of the sensor module 50 are made to simultaneously emit light so that the skin surface of the user 2 is irradiated with measurement light. In addition, all of the light receiving elements 59 are used to receive light (transmitted light) which is a result of the measurement light being transmitted through the biological tissue or light (reflected light) which is a result of the measurement light being reflected in the biological tissue so as to capture an image of the biological tissue, thereby acquiring a biological image.

Fig. 4 is a diagram illustrating an example of a biological image P2. The biological image P2 can be obtained as a two-dimensional image having the same number of pixels as in the arrangement of the light receiving elements 59 of the sensor module 50.

Since the blood vessel more easily absorbs near infrared rays than a non-blood vessel portion, the blood vessel portion has lower luminance and is darker than the non-blood vessel portion in the biological image P2. For this reason, the portion having the low luminance in the biological image P2 is extracted, and thus a blood vessel pattern can be extracted. In other words, on the basis of whether or not the luminance of each pixel forming the biological image P2 is equal to or lower than a predetermined threshold value, the presence or absence of a blood vessel directly under the light receiving elements 59, that is, a position of the blood vessel can be acquired.

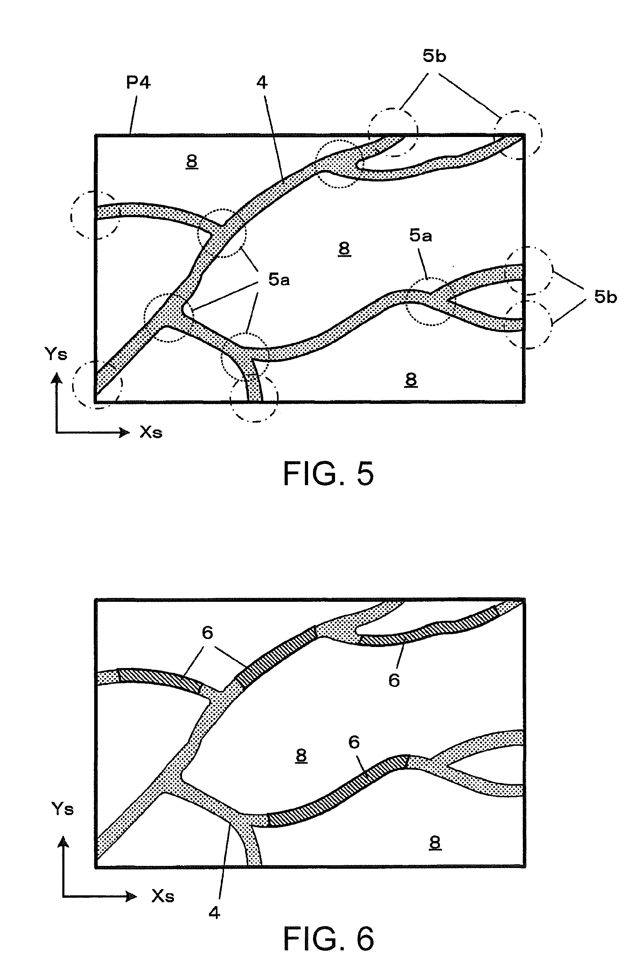

Fig. 5 is a diagram illustrating an example of a blood vessel pattern P4 obtained on the basis of the biological image P2 of Fig. 4. The blood vessel pattern P4 is information indicating whether a corresponding region is a blood vessel region or a non-blood vessel region for each pixel forming the biological image, that is, for each position of the light receiving element 59. In Fig. 5, a shaded strip-shaped portion is a blood vessel 4, and the other white portion is extracted as a non-blood vessel region 8.

### (A-2) Selection of measurement target blood vessel part

If the blood vessel pattern is acquired, then, a measurement target blood vessel (more specifically, a blood vessel part) is selected. A measurement target blood vessel part 6 is selected so as to satisfy the following selection condition. The selection condition is that "the blood vessel part is a part other than a branch portion or a joint portion of the blood vessel and an end portion of the image, and has a predetermined length and a predetermined width in a blood vessel length direction".

In branch or joint portions 5a (refer to Fig. 5) of the blood vessel, light which has passed through a blood vessel which is not a measurement target may be mixed with received light. The light which has passed through the blood vessel other than the measurement target blood vessel part 6 may influence an absorption spectrum of the measurement target blood vessel part 6 and thus measurement accuracy may decrease. For this reason, the measurement target blood vessel part 6 is selected from blood vessel portions excluding the branch or joint portions 5a of the blood vessel.

In end portions 5b (refer to Fig. 5) of the biological image, a structure such as branch or joint of the blood vessel near the outside of the image is unclear, and thus measurement accuracy may decrease for the above-described reason. In order to prevent this situation, the measurement target blood vessel part 6 is selected from blood vessel portions excluding the image end portions 5b.

In addition, irradiation light from the light emitting elements 53 is diffused and reflected inside a biological tissue, and some of the reflected light is received by the light receiving elements 59. In other words, some of the light received by the light receiving element 59 becomes transmitted light of the target blood vessel, but as a ratio of the transmitted light increases, an absorption spectrum which more remarkably represents a feature of a blood component of the target blood vessel may be obtained. In other words, measurement accuracy increases.

In addition, a relatively thinly reflected blood vessel (a short blood vessel in the width direction) is an originally thin blood vessel, and is a blood vessel which is located at a relatively deep position. An amount of transmitted light is small in this blood vessel, and thus measurement accuracy may decrease. For this reason, the measurement target blood vessel part 6 is selected from blood vessel portions (that is, a blood vessel part having a predetermined width) excluding the thinly reflected blood vessel.

Fig. 6 is a diagram illustrating an example of the measurement target blood vessel part 6 obtained on the basis of the blood vessel pattern P4 of Fig. 5. In Fig. 6, a hatched portion of the blood vessel 4 is the blood vessel part 6 which is selected as a measurement target.

### (A-3) Selection of irradiation position and light reception position

Next, in relation to the selected measurement target blood vessel part 6, an irradiation position (measurement light emitting element) of measurement light, an light reception position (measurement light receiving element) which is suitable for receiving transmitted light of the measurement target blood vessel part 6, and a light reception position (reference light receiving element) which is suitable for obtaining reference transmitted light, are selected. The reference transmitted light is light which is not transmitted through the measurement target blood vessel part 6 but is transmitted only through the non-blood vessel region 8 near the blood vessel part 6.

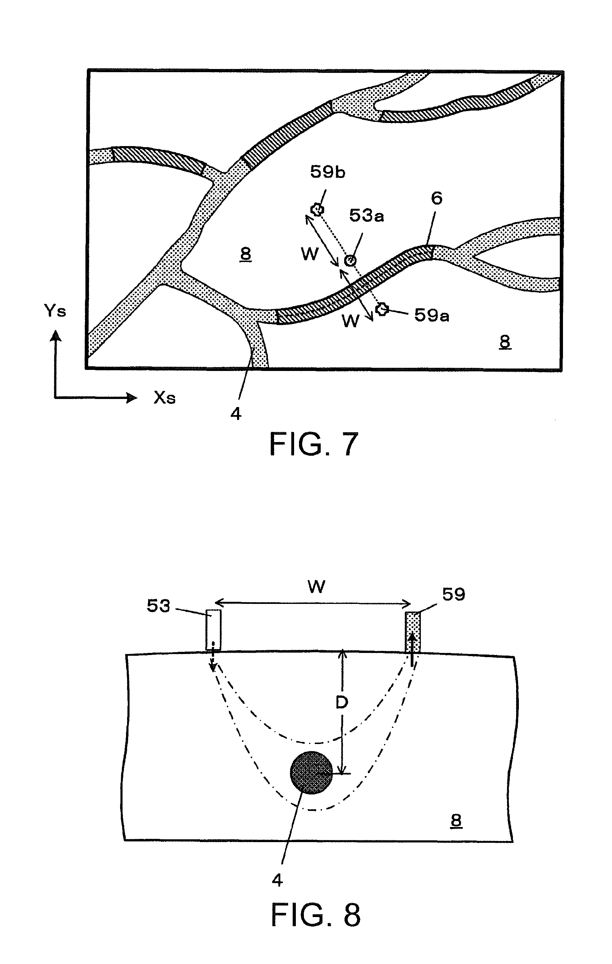

Fig. 7 is a diagram for explaining selection of the irradiation position, the measurement light reception position, and the reference light reception position. First, the irradiation position (measurement light emitting element) and the measurement light reception position (measurement light receiving element) are selected so as to satisfy the following first relative position condition. The first relative position condition is that, in the blood vessel pattern, "the measurement target blood vessel part 6 is located at a center between the irradiation position and the measurement light reception position and a distance between the irradiation position and the measurement light reception position is the same as a predetermined optimum distance W". The light emitting element 53 at the irradiation position satisfying the first relative position condition is set as a measurement light emitting element 53a, and the light receiving element 59 at the measurement light reception position is set as a measurement light receiving element 59a.

In addition, the irradiation position (measurement light emitting element) and the reference light reception position (reference light receiving element) are selected so as to satisfy the following second relative position condition. The second relative position condition is that, in the blood vessel pattern, "there is no blood vessel between the irradiation position and the reference light reception position and a distance between the irradiation position and the reference light reception position is the same as the predetermined optimum distance W". The light receiving element 59 at the reference light reception position satisfying the second relative position condition is set as a reference light receiving element 59b.

In the present embodiment, the reference light reception position is located on an extension of the line connecting the measurement irradiation position and the measurement light reception position to each other satisfying the above-described first relative position condition and is located opposite to the measurement light reception position when viewed from the measurement irradiation position, but is not limited thereto. It is assumed that none of the measurement irradiation position (the measurement light emitting element 53a), the measurement light reception position (the measurement light receiving element 59a), and the reference light reception position (reference light receiving element) are located over the blood vessel 4 (the positions are located in the non-blood vessel region 8).

The optimum distance W is defined as follows. Fig. 8 is a diagram for explaining propagation of light in a biological tissue, and is a cross-sectional view in a depth direction. Light applied from the light emitting element 53 is diffused and reflected inside the biological tissue, and some of the applied light reaches a certain light receiving element 59. A propagation path of the light forms a so-called banana shape (a region interposed between two arcs), a width in the depth direction is largest in the vicinity of approximately a center thereof, and the entire depth (reachable depth) is increased in accordance with a gap between the light emitting element 53 and the light receiving element 59.

If measurement accuracy is to be increased, it is desirable that an amount of transmitted light which is greater than an amount of light transmitted through the blood vessel 4 is received by the light receiving element 59. From this factor, the target blood vessel 4 is located nearly at a center between the light emitting element 53 and the light receiving element 59, and the optimum distance W is defined according to an expected depth D of the target blood vessel 4. The optimum distance W, that is, the optimum gap W between the light emitting element 53 and the light receiving element 59 is a distance which approximately doubles a depth D of the blood vessel 4 from the skin surface. For example, if the depth D is about 3 mm, the optimum distance W is about 5 mm to 6 mm.

### (A-4) Measurement

If the irradiation position and the light reception position are determined for the measurement target blood vessel part 6, a blood glucose level is measured. In other words, measurement light is applied from the measurement irradiation position (the measurement light emitting element 53a) set for the measurement target blood vessel part 6, and an absorption spectrum is generated on the basis of each light reception result at the measurement light reception position (the measurement light receiving element 59a) and the reference light reception position (the reference light receiving element 59b).

In this case, for example, a wavelength of light emitted by the light emitting element 53 is changed so that a wavelength λ of light applied to the skin surface is changed within a near infrared region, and thus the transmittance of the blood vessel part 6 is obtained for each wavelength λ. The transmittance T (λ) is obtained as T (λ) = Os (λ) /Or (λ) on the basis of light intensity Os(λ) obtained by the measurement light receiving element 59a and light intensity Or(λ) obtained by the reference light receiving element 59b. An absorption ratio is obtained on the basis of the transmittance, and then an absorption spectrum is generated.

Here, a calculation principle of the transmittance R will be described briefly. Generally, when the intensity of light applied by the light emitting element 53 is P(λ), the transmittance of an object portion through which the irradiation light is transmitted is T(λ), and the sensitivity defined for the light receiving element 59 is S(λ), a light intensity O(λ) obtained by the light receiving element 59 is given as O(λ) =P(λ)·T(λ)·S(λ).

From this relational expression, the light intensity Or (λ) obtained by the reference light receiving element 59b, not including transmitted light of the blood vessel 4 becomes Or (λ) =P(λ)·T(λ)·S(λ) assuming that the transmittance T(λ) of the non-blood vessel region portion is "1".

In addition, the light intensity Os(λ) obtained by the measurement light receiving element 59a, including transmitted light of the blood vessel 4 becomes Os (λ) =P(λ)·T(λ)·S(λ). From the two equations, the transmittance T(λ) is obtained. The transmittance T(λ) is a relative value for the transmittance of the non-blood vessel region 8.

### (A-5) Calculation of blood glucose level

Next, on the basis of the absorption spectrum, a blood glucose level is estimated and calculated by using a calibration curve indicating a relationship between a predefined blood glucose level (a glucose concentration in blood) and an absorbance. In addition, a technique for calculating a concentration of a predetermined component (glucose in the present embodiment) on the basis of the absorption spectrum is well known, and the well-known technique may be employed in the present embodiment.

### (B) Partial light emission

In the present embodiment, when a blood glucose level is measured and a biological image is acquired, all of the light emitting elements 53 of the sensor module 50 do not emit light but only some of the light emitting elements 53 emit light, and thus power saving is further realized. The light emitting elements 53 which are made to emit light during the acquisition of a biological image are determined on the basis of an irradiation position (the measurement light emitting element 53a) in the previous measurement.

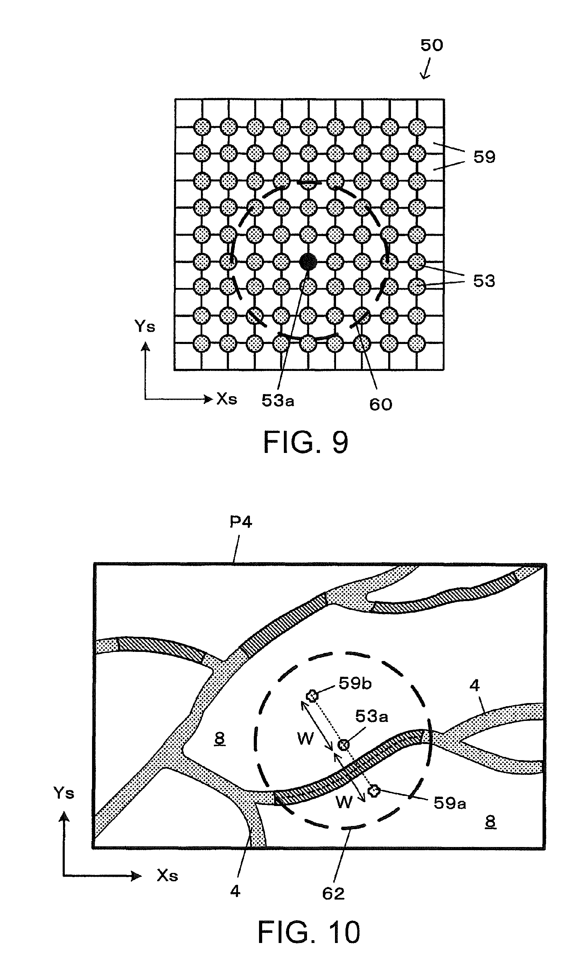

Fig. 9 is a diagram for explaining selection of the light emitting element 53 which is made to emit light during the acquisition of a biological image, and is a plan view of the sensor module 50. As illustrated in Fig. 9, a light emission range 60 which is a circular range with a predetermined radius R is defined centering on the measurement light emitting element 53a in the previous measurement, and only the light emitting elements 53 in this light emission range 60 are made to emit light.

The radius R may be set to be longer than the optimum distance W which is used to set, for example, the measurement light reception position and the reference light reception position. Since the blood glucose level measurement apparatus 10 of the present embodiment is mounted on the wrist or the like of the user 2 and is used, there is a high possibility that a relative positional relationship between the sensor module 50 and the skin surface of the user 2 may not be changed much from the previous measurement. For this reason, in a case where the light emission range 60 is defined as described above, there is a high possibility that the measurement target blood vessel part 6 in the previous measurement may be included in a range 62 (imaging range) of an acquired biological image as illustrated in Fig. 10.

Since the radius R is defined to be longer than the optimum distance W, the imaging range 62 includes the measurement target blood vessel part 6 (specifically, the blood vessel part 6 interposed between the irradiation position (the measurement light emitting element 53a) and the measurement light reception position (the measurement light receiving element 59a)) in the previous measurement.

In a case where a mounting position of the blood glucose level measurement apparatus 10 is deviated due to remounting or the like, the blood vessel 4 may not be included in an acquired biological image, that is, the blood vessel pattern may not be acquired. In this case, the radius R is reset to be long so as to widen the light emission range 60, and a biological image may be acquired again.

### Functional Configuration

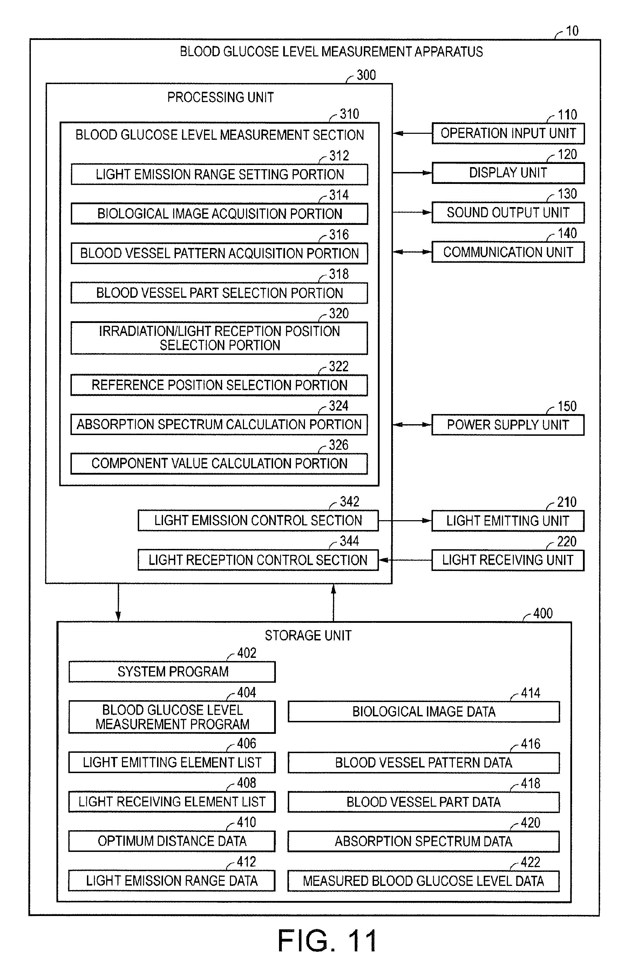

Fig. 11 is a functional configuration diagram of a blood glucose level measurement apparatus 10A in the present embodiment. The blood glucose level measurement apparatus 10 functionally includes an operation input unit 110, a display unit 120, a sound output unit 130, a communication unit 140, a light emitting unit 210, a light receiving unit 220, a processing unit 300, and a storage unit 400.

The operation input unit 110 is an input device such as a button switch, a touch panel, or various sensors, and outputs an operation signal corresponding to a performed operation to the processing unit 300. Various instruction inputs such as a measurement start instruction of a blood glucose level are performed via the operation input unit 110. In Fig. 1, the operation switch 18 or the touch panel 16 corresponds to the operation input unit 110.

The display unit 120 is a display device such as a liquid crystal display (LCD), and performs various displays based on display signals from the processing unit 300. A measurement result and the like are displayed on the display unit 120. In Fig. 1, the touch panel 16 corresponds to the display unit 120.

The sound output unit 130 is a sound output device such as a speaker, and outputs various sounds based on sound signals from the processing unit 300. The sound output unit 130 outputs alarm sounds of measurement start or measurement end of a blood glucose level, the occurrence of a low blood glucose level, and the like.

The communication unit 140 is a communication device such as a wireless communication device, a modem, a wired communication cable jack, or a control circuit, and performs communication with external devices through connection to a communication line. In Fig. 1, the communication device 20 corresponds to the communication unit 140.

The light emitting unit 210 includes a plurality of light emitting elements 53 which are arranged in the planar shape in a two-dimensional manner. The light emitting layer 52 of the sensor module 50 illustrated in Figs. 2A and 2B corresponds to the light emitting unit 210. An arrangement position of the light emitting unit 210 (specifically, position coordinates of each light emitting element 53 in the Xs-Ys orthogonal coordinate system) is stored as a light emitting element list 406.

The light receiving unit 220 includes a plurality of light receiving elements 59 which are arranged in the planar shape in a two-dimensional manner. The light receiving layer 58 of the sensor module 50 illustrated in Figs. 2A and 2B corresponds to the light receiving unit 220. An arrangement position of the light receiving unit 220 (specifically, position coordinates of each light receiving element 59 in the Xs-Ys orthogonal coordinate system) is stored as a light receiving element list 408.

The processing unit 300, which is implemented by, for example, a microprocessor such as a CPU or a graphics processing unit (GPU), or an electronic part such as an application specific integrated circuit (ASIC) or an IC memory, performs various calculation processes on the basis of a predetermined program or data or an operation signal from the operation input unit 110, and controls an operation of the blood glucose level measurement apparatus 10. In Fig. 1, the control board 30 corresponds to the processing unit 300. In addition, the processing unit 300 includes a blood glucose level measurement section 310, a light emission control section 342, and a light reception control section 344.

The blood glucose level measurement section 310 includes a light emission range setting portion 312, a biological image acquisition portion 314, a blood vessel pattern acquisition portion 316, a blood vessel part selection portion 318, an irradiation/light reception position selection portion 320, a reference position selection portion 322, an absorption spectrum calculation portion 324, and a component value calculation portion 326, and measures a glucose concentration in blood of the user 2, that is, a blood glucose level.

The light emission range setting portion 312 sets the light emission range 60 of the light emitting elements 53 in the light emitting unit 210 when a biological image is acquired, on the basis of measurement information in a past measurement. In other words, in the light emission surface of the light emitting unit 210, the light emission range 60 which is a circular range with the predetermined radius R centering on an irradiation position (the measurement light emitting element 53a) in the previous measurement is set. The irradiation position (the measurement light emitting element 53a) in the previous measurement is included in blood vessel part data 418. In addition, the light emission range 60 set by the light emission range setting portion 312 is stored as light emission range data 412.

Here, in a case where there are a plurality of irradiation positions (the measurement light emitting elements 53a) in the previous measurement, a plurality of light emission ranges 60 which respectively correspond to the plurality of irradiation positions, or light emission ranges 60 which respectively correspond to one or a plurality of irradiation positions selected from among the plurality of irradiation positions may be set. In a case of an initial measurement, there is no irradiation position in the previous measurement, and thus a range (that is, the entire light emission surface of the light emitting unit 210) including all the light emitting elements 53 is set as the light emission range 60.

The biological image acquisition portion 314 acquires a biological image of the user 2. The acquisition of a biological image is performed by appropriately using a biological image capturing technique in the well-known vein authentication technique or the like. In other words, among the light emitting elements 53 of the light emitting unit 210, the light emitting elements 53 within the light emission range 60 set by the light emission range setting portion 312 simultaneously emit light, and all the light receiving elements 59 perform photometry (perform imaging). A luminance image based on a photometry result, that is, a biological image is generated. The biological image acquired by the biological image acquisition portion 314 is stored as biological image data 414.

The blood vessel pattern acquisition portion 316 performs a predetermined image process on the biological image acquired by the biological image acquisition portion 314, so as to acquire a blood vessel pattern. Specifically, the acquisition is performed by appropriately using a technique for identifying a vein pattern from a biological image in the well-known vein authentication technique. For example, a binarization or a filtering process is performed on each pixel of the biological image through comparison with reference luminance. A pixel having luminance lower than the reference luminance indicates a blood vessel region, and a pixel having luminance equal to or higher than the reference luminance indicates a non-blood vessel region. The blood vessel pattern acquired by the blood vessel pattern acquisition portion 316 is stored as blood vessel pattern data 416.

The blood vessel part selection portion 318 selects the blood vessel part 6 satisfying a predetermined selection condition as a measurement target on the basis of the blood vessel pattern acquired by the blood vessel pattern acquisition portion 316. Here, the blood vessel part 6 may be selected alone or in a plurality as a measurement target. Each blood vessel part 6 selected as a measurement target is stored as blood vessel part data 418.

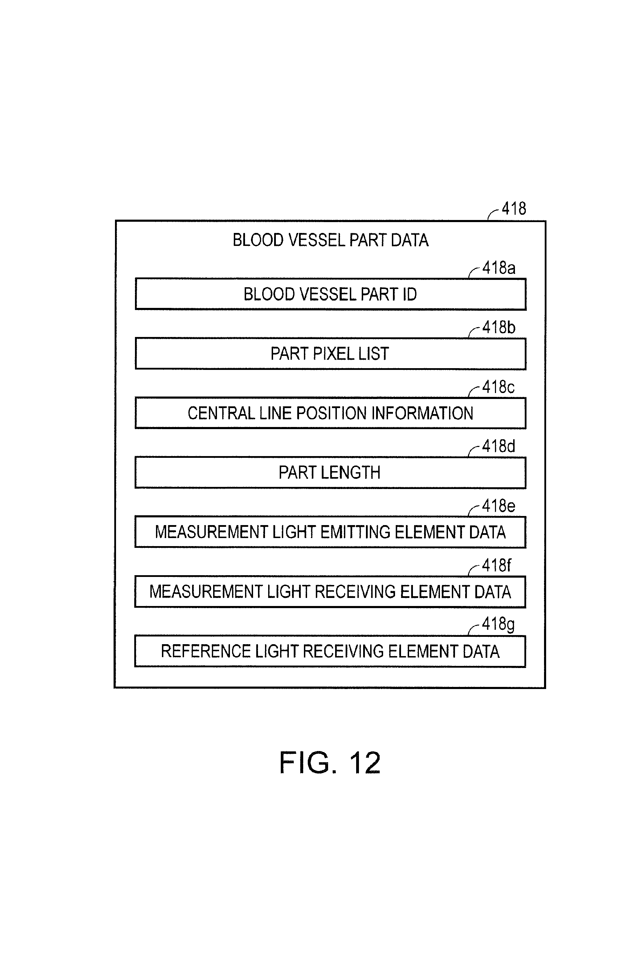

Fig. 12 is a diagram illustrating an example of a configuration of the blood vessel part data 418. The blood vessel part data 418 stores a blood vessel part ID 418a which is identification information of the corresponding blood vessel part, a part pixel list 418b, central line position information 418c, a part length 418d which is a length in the blood vessel length direction, measurement light emitting element data 418e, measurement light receiving element data 418f, and reference light receiving element data 418g. The part pixel list 418b is a list of pixels (that is, the light receiving elements 59) corresponding to the blood vessel part. The central line position information 418c is information on position coordinates of a central line (which is a center in the blood vessel width direction and is a line in the blood vessel length direction) of the blood vessel part in the Xs-Ys orthogonal coordinate system.

The irradiation/light reception position selection portion 320 selects an irradiation position (the measurement light emitting element 53a) and a measurement light reception position (the measurement light receiving element 59a) so that each measurement target blood vessel part 6 satisfies the first relative position condition. Specifically, the irradiation position and the measurement light reception position are set to two positions, with the blood vessel part 6 interposed therebetween, which have the optimum distance W as a gap therebetween in two directions perpendicular to the central line from a certain position on the central line of the blood vessel part 6 in the Xs-Ys orthogonal coordinate system (that is, in the skin surface). In addition, the light emitting element 53 at the irradiation position is used as the measurement light emitting element 53a, and the light receiving element 59 at the measurement light reception position is used as the measurement light receiving element 59a. The optimum distance W is stored as optimum distance data 410. As a method of selecting a certain position on the central line, an approximately central position of the blood vessel part 6 in the length direction is defined.

In a case where there are no positions (that is, positions which can be set as the irradiation position and the measurement light reception position) satisfying the first relative position condition for the selected certain position, it is similarly determined whether or not there are positions (that is, positions which can be set as the irradiation position and the measurement light reception position) satisfying the first relative position condition for a position far from the certain position along the central line by a predetermined unit distance. In a case where positions satisfying the first relative position conditions are not found yet, the process is repeatedly performed in the same manner, and the measurement irradiation position and the measurement light reception position are searched for and set.

The reference position selection portion 322 selects a reference light reception position (the reference light receiving element 59b) so as to satisfy the second relative position condition with respect to the irradiation position and the measurement light reception position set by the irradiation/light reception position selection portion 320. In addition, in a case where there is no position satisfying the second relative position condition, the irradiation position and the measurement light reception position are searched for and set again by the irradiation/light reception position selection portion 320.

The absorption spectrum calculation portion 324 generates an absorption spectrum for each measurement target blood vessel part 6. Specifically, the absorption spectrum is generated by calculating the transmittance T for each wavelength λ on the basis of a light reception result (light intensity) obtained from the measurement light receiving element 59a and the reference light receiving element 59b set for the blood vessel part 6. In addition, in a case where there are a plurality of measurement target blood vessel parts 6, respective absorption spectra of the plurality of measurement target blood vessel parts 6 are averaged, and thus an average absorption spectrum is calculated. The absorption spectrum calculated by the absorption spectrum calculation portion 324 is stored as absorption spectrum data 420.

The component value calculation portion 326 calculates a glucose concentration (that is, a blood glucose level) which is a concentration in blood of an aimed blood component on the basis of the absorption spectrum calculated by the absorption spectrum calculation portion 324. In the present embodiment, the absorption spectrum is obtained by using an analysis method such as a multiple regression analysis method, a main component regression analysis method, a PLS regression analysis method, or an independent component analysis method. In a case where there are a plurality of measurement target blood vessel parts 6, a blood glucose level is calculated on the basis of an average absorption spectrum which is obtained by averaging absorption spectra related to the respective blood vessel parts 6. The blood glucose level calculated by the component value calculation portion 326 is accumulated and stored as measured blood glucose level data 422 in correlation with the measurement time.

The light emission control section 342 selectively controls light emission of each of the plurality of light emitting elements 53 of the light emitting unit 210. The light reception control section 344 acquires an amount of received light from each of the plurality of light receiving elements 59 of the light receiving unit 220.

The storage unit 400, which is a storage device such as a ROM, a RAM, or a hard disk, stores a program, data, or the like for the processing unit 300 collectively controlling the blood glucose level measurement apparatus 10, and is used as a work area of the processing unit 300 so as to temporarily store a result of calculation performed by the processing unit 300 or operation data from the operation input unit 110. In Fig. 1, the main memory or the measurement data memory mounted on the control board 30 corresponds to the storage unit 400. The storage unit 400 stores a system program 402, a blood glucose level measurement program 404, the light emitting element list 406, the light receiving element list 408, the optimum distance data 410, the light emission range data 412, the biological image data 414, the blood vessel pattern data 416, the blood vessel part data 418, the absorption spectrum data 420, and the measured blood glucose level data 422.

### Flow of Process

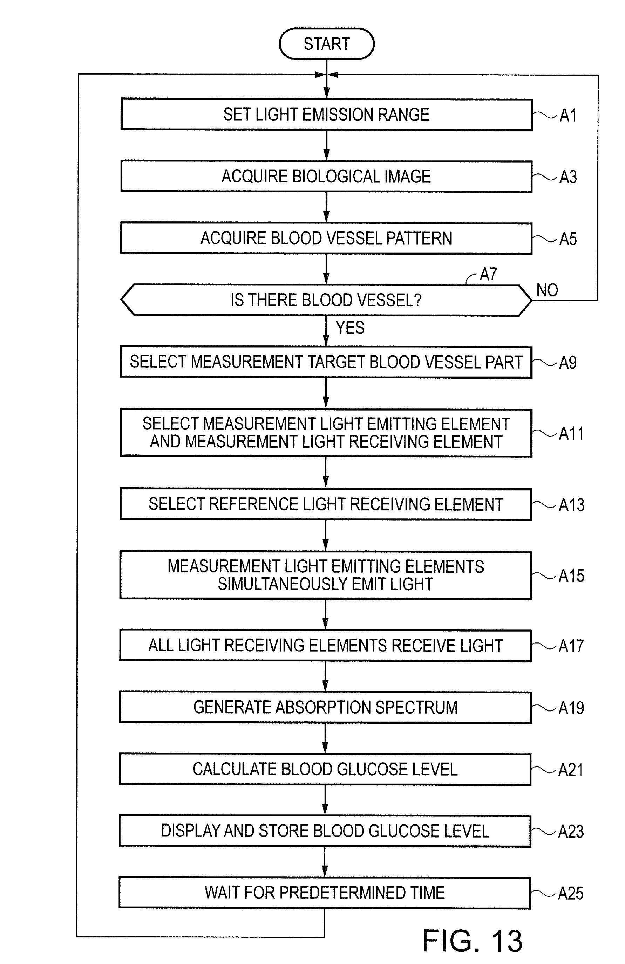

Fig. 13 is a flowchart illustrating a flow of a blood glucose level measurement process. This process is realized by the processing unit 300 performing a process according to the blood glucose level measurement program 404.

In Fig. 13, first, the blood glucose level measurement section 310 performs a measurement process of measuring a blood glucose level of the user. In other words, the light emission range setting portion 312 sets the light emission range 60 of the sensor module 50 (step A1). That is, in a first measurement, the entire light emission surface (that is, a range including all the light emitting elements 53) of the sensor module 50 is set as the light emission range 60, and, in the second and subsequent measurements, a range centering on an irradiation position (the measurement light emitting element 53a) in the previous measurement is used as the light emission range 60.

Next, among the light emitting elements 53 of the sensor module 50, only the light emitting elements 53 in the set light emission range 60 are made to emit light, and thus the biological image acquisition portion 314 acquires a biological image of the user (step A3). Next, the blood vessel pattern acquisition portion 316 acquires a blood vessel pattern which is viewed from the skin surface on the basis of the obtained biological image (step A5). If a blood vessel pattern cannot be obtained (step A7: NO), the flow returns to step A1, and the light emission range 60 is reset (step A1). At this time, for example, the light emission range 60 is reset to be widened by increasing the radius R by a predetermined length ΔR. In addition, the light emitting elements 53 within the reset light emission range 60 are made to emit light so that a biological image is acquired again (step A3), and a blood vessel pattern is acquired again on the basis of the biological image which is acquired again (step A5).

If the blood vessel pattern is obtained (step A7: YES), the blood vessel part selection portion 318 selects the measurement target blood vessel part 6 satisfying a predetermined selection condition on the basis of the obtained blood vessel pattern (step A9). The irradiation/light reception position selection portion 320 selects an irradiation position (the measurement light emitting element 53a) and a measurement light reception position (the measurement light receiving element 59a) satisfying the first relative position condition for each measurement target blood vessel part 6 (step A11). Next, the reference position selection portion 322 selects a reference light reception position (the reference light receiving element 59b) satisfying the second relative position condition for each measurement target blood vessel part 6 (step A13).

Next, the measurement light emitting elements 53a set for the respective measurement target blood vessel parts 6 simultaneously emit light (step A15), and all the light receiving elements 59 receive light (perform imaging) (step A17). Next, the absorption spectrum calculation portion 324 generates an absorption spectrum for the blood vessel part 6 on the basis of a light reception result (light intensity) obtained from the respective measurement light receiving elements 59a and reference light receiving elements 59b set for each measurement target blood vessel part 6. In addition, in a case where there are a plurality of measurement target blood vessel parts 6, an absorption spectrum is calculated by averaging absorption spectra for the respective blood vessel parts 6 (step A19). Then, the component value calculation portion 326 calculates a glucose concentration in blood, that is, a blood glucose level on the basis of the absorption spectrum (step A21). The calculated blood glucose level is displayed on the display unit 120, and is accumulated and stored in correlation with the measurement time (step A23).

A wait occurs while the predetermined time elapses (step A25), and the flow returns to step A1, and the next measurement of a blood glucose level is performed in the same manner.

### Operations and Effects

As mentioned above, according to the present embodiment, when a biological image is captured in order to acquire a blood vessel pattern, all of the light emitting elements 53 do not emit light (entire surface emission is not performed) but only some of the light emitting elements 53 in the light emission range 60 emit light, and thus power consumption related to a measurement can be reduced. The light emission range 60 is set to a range centering on an irradiation position (the measurement light emitting element 53a) in the previous measurement. Since the blood glucose level measurement apparatus 10 is mounted on the wrist or the like of the user 2 and is used, there is a high possibility that a relative positional relationship between the sensor module 50 and the skin surface of the user 2 may not be changed much from the previous measurement. For this reason, there is a high possibility that a blood vessel (the measurement target blood vessel part 6 in the previous measurement) may be included in a range of an acquired biological image.

### Modification Examples

An applicable embodiment of the invention is not limited to the above-described embodiment and may include modifications within the scope not departing from the spirit of the invention as appropriate.

### (A) Shape of light emission range 60

In the above-described embodiment, a light emission range has a circular shape but is not limited thereto, and may have other shapes such as a rectangular shape.

### (B) Resetting of light emission range 60

In the above-described embodiment, in a case where a blood vessel pattern cannot be acquired from a biological image, the light emission range 60 is reset to be widened, but the light emission range 60 may be set at a different position. Specifically, for example, a new light emission range 60 is reset so as not to overlap the preset light emission range 60. This is because there is a high possibility that a mounting position of the blood glucose level measurement apparatus 10 may be deviated due to remounting or the like.

### (C) Light emission range 60 during initial measurement

In the above-described embodiment, a range (that is, the entire emission surface of the light emitting unit 210) including all of the light emitting elements 53 is set as the light emission range 60 during the initial measurement, but a range during the initial measurement may be set to be wider than the light emission range 60 (a circular range with the radius R) set in the second and subsequent measurements, and may be a range of 80% of the entire emission range.

### (D) Biological information

In the above-described embodiment, a concentration of glucose (that is, a blood glucose level) which is a component in blood is measured as biological information related to a blood vessel or the blood, but the invention is also applicable to a case where different blood components are measured or a case where not a blood component but a blood vessel itself (for example, a blood vessel diameter) is detected.

## Claims

1. A biological information measurement apparatus comprising:
an optical sensor unit that includes a light emitting unit in which a plurality of light emitting elements are disposed in a planar shape, and a light receiving unit receiving light which is a result of light emitted by the light emitting unit being reflected or transmitted inside a living body;
a light emission range setting unit that sets a light emission range of the light emitting unit;
a blood vessel position detecting unit that causes the light emitting elements within the light emission range to emit light and detects a blood vessel position on the basis of an image obtained by the light receiving unit receiving the light; and
a measurement unit that controls light emission of the light emitting elements selected on the basis of the detected blood vessel position, and light reception in the light receiving unit, so as to measure biological information related to a blood vessel or blood,
wherein the light emission range setting unit sets the light emission range on the basis of past measurement information obtained by the measurement unit.

2. The biological information measurement apparatus according to claim 1, wherein the light emission range setting unit sets the light emission range on the basis of a position of a light emitting element which is selected in a past measurement by the measurement unit.

3. The biological information measurement apparatus according to claim 1 or claim 2, wherein, in a case where there is no past measurement performed by the measurement unit, the light emission range setting unit sets, as the light emission range, a range which is wider than in a case where there is a past measurement.

4. The biological information measurement apparatus according to any of claims 1-3, further comprising:
a re-execution control unit that causes the light emission range setting unit to reset a different range as the light emission range and the blood vessel position detecting unit to detect a blood vessel position again in a case where a blood vessel position is not detected by the blood vessel position detecting unit.

5. The biological information measurement apparatus according to claim 4, wherein the re-execution control unit causes the light emission range setting unit to reset a range which is wider than a previously set light emission range, as the light emission range.

6. A biological information measurement method comprising:
setting a light emission range of a light emitting unit of an optical sensor unit that includes the light emitting unit in which a plurality of light emitting elements are disposed in a planar shape, and a light receiving unit receiving light which is a result of light emitted by the light emitting unit being reflected or transmitted inside a living body;
causing the light emitting elements within the light emission range to emit light and detecting a blood vessel position on the basis of an image obtained by the light receiving unit receiving the light; and
controlling light emission of the light emitting elements selected on the basis of the detected blood vessel position, and light reception in the light receiving unit, so as to measure biological information related to a blood vessel or blood,
wherein the setting of the light emission range includes setting the light emission range on the basis of past measurement information obtained by measuring the biological information.
